# FASCICULE DE BREVET EUROPEEN

(11) **EP 1 412 462 B1**
(45) Date de publication et mention de la délivrance du brevet: **15.04.2020**
(21) Numéro de dépôt: 02762506.0
(22) Date de dépôt: 28.06.2002
(51) Int. Cl.: C11C 3/00, A61K 47/00, C10M 159/00, C09D 11/00, A61Q 1/06, A61Q 19/00, A61K 8/92, A61K 9/00, A61K 9/107, A61K 47/44, C10M 159/12, C11C 3/02, C09G 1/14, C14C 9/02

(54) **SUBSTITUT DE LANOLINE SON PROCEDE D OBTENTION ET SES APPLICATIONS**
LANOLINERSATZSTOFF, HERSTELLUNGSVERFAHREN DAFÜR UND ANWENDUNGEN DAVON
LANOLIN SUBSTITUTE, PRODUCTION METHOD THEREOF AND APPLICATIONS OF SAME

(30) Priorité: 02.07.2001 FR 0108905
(43) Date de publication de la demande: 28.04.2004
(60) Demande divisionnaire: 18192788.0
(73) Titulaire: Aldivia S.A., 69561 Saint-Genis Laval Cedex (FR)
(72) Inventeur: CHARLIER DE CHILY, Pierre, F-60540 Irigny (FR); RAYNARD, Mikaele, F-44250 SAINT-BREVIIN-LES-PINS (FR)
(74) Mandataire: Lavoix
(86) Numéro de dépôt international: PCT/FR2002/002271
(87) Numéro de publication internationale: WO 2003/004590

(56) Documents cités:
- WO-A-00/26265
- WO-A-92/17434
- WO-A-98/04597
- US-A- 1 728 205
- US-A- 2 098 551
- US-A- 4 428 885
- US-A- 5 436 006
- PATENT ABSTRACTS OF JAPAN vol. 013, no. 278 (C-611), 26 juin 1989 (1989-06-26) & JP 01 074298 A (NIPPON OIL & FATS CO LTD), 20 mars 1989 (1989-03-20)
- CHEMICAL ABSTRACTS, vol. 96, no. 26, 28 juin 1982 (1982-06-28) Columbus, Ohio, US; abstract no. 223306n, VERBUTA, ANETA ET AL.: "Glyceridic emulsidier as a substitute for lanolin and other surfactants" page 380; colonne 1; XP002195174 & RO 70 144 A (INTREPRINDEREA DE SAPUN "STELA") 8 avril 1980 (1980-04-08)
- CHEMICAL ABSTRACTS, vol. 95, no. 14, 5 octobre 1981 (1981-10-05) Columbus, Ohio, US; abstract no. 117408s, HIGAKI, YUZO: "Paste ester hydrate" page 105; colonne 1; XP002195175 & FUREGURANSU JANARU, vol. 9, no. 1, 1981, pages 51-55,
- PATENT ABSTRACTS OF JAPAN vol. 006, no. 024 (C-091), 12 février 1982 (1982-02-12) & JP 56 145208 A (SHISEIDO CO LTD), 11 novembre 1981 (1981-11-11)

## Description

### Secteur technique de l'invention et problème posé :

La lanoline, graisse extraite du suint du mouton, est couramment utilisée par les industries cosmétiques et pharmaceutiques pour ses propriétés émolliente, hydratante, émulsionnante et sa capacité d'absorption en eau.

En dépit de toutes ces propriétés, l'origine animale de la lanoline satisfait de moins en moins le consommateur, rendu inquiet par la tremblante du mouton. Les réactions allergiques provenant de résidus pesticides, sa forte odeur, sa composition variable, son irrégularité d'approvisionnement, etc, sont des facteurs supplémentaires qui poussent les scientifiques à chercher des substituts de lanoline.

Les tentatives de substitution de la lanoline par des produits oléochimiques sont nombreuses.

La première solution envisagée consiste à recréer la formule de la lanoline en excluant des ingrédients d'origine animale. La complexité de la composition de la lanoline rend cette solution difficile : les coûts occasionnés sont trop onéreux pour continuer dans cette voie.

Une deuxième solution pour pallier ce problème consiste à fabriquer un produit de composition différente à celle de la lanoline mais aux propriétés semblables.

Jusqu'à présent, les substituts proposés n'apportent pas entière satisfaction de par leurs propriétés, la complexité de leur composition, certaines caractéristiques physico-chimiques élevées comme l'indice d'acide, et/ou leur coût de fabrication.

### Art antérieur :

### La lanoline

### • Composition de la lanoline :

La lanoline ou graisse de laine est fabriquée à partir des sécrétions sébacées de la graisse de mouton. C'est un mélange de stérols et d'alcools gras à longues chaînes. Ce mélange contient parfois d'autres substances (nickel, chrome, cuivre...) sous forme de traces.

Sa composition est complexe comme le montrent les tableaux suivants :

**Tableau n°1 : Composition de la lanoline (% dans la cire)**

| **Constituents** | **%** | **Constituents après saponification** | **%** |
|---|---|---|---|
| Acides libres | 0-1 | Acides gras | 29-38 |
| Esters d'alcools triterpéniques et de stérols | 29-44 | α-hydroxyacides | 11-15 |
| Esters d'alcools aliphatiques | 14-24 | β-hydroxyacides | 1-3 |
| Monohydroxyesters de stérols, alcools aliphatiques et triterpéniques | 15-20 | Polyhydroxyacides | 3-6 |
| Alcools libres aliphatiques | 6-20 | Alcools aliphatiques | 5-15 |
| Alcools triterpéniques et stérols libres | 4-5 | α- diols | 4-6 |
| Di et polyhydroxy esters et diols libres | 7-9 | Polyols | 3-8 |
| Dérivés d'oxydation | 2-5 | Cholestérol | 11-20 |
| Hyd rocarbu res | 0-1 | Lanostérol | 1-8 |
| | | Dihydrolanosterol | 1-5 |
| | | 7-kétocholesta-3,5-diène | 2-4 |
| | | 3-β-hydroxy-7-kéta-lanosta-8ène | 1-4 |
| | | Dérivés d'oxydation | 2-5 |
| | | Hydrocarbures | 0-1 |
| T = trace | | | |
| n = linéaire | | | |
| i = isomère | | | |
| ai = anté-isomère | | | |

**Tableau n°2 : Composition des chaines carbonées de la lanoline (% de chaque constituant)**

| **Chaine** | **Isomères** | **Acides gras** | **α-hydroxyacides** | **Alcools gras** | **1.2 alcane diol** |
|---|---|---|---|---|---|
| C10 | n | T | T | - | - |
| | i | T | - | - | - |
| C11 | n | T | T | - | - |
| | ai | 0t1 | - | - | - |
| C12 | n | T | T | T | T |
| | i | 0-1 | T | T | - |
| C13 | n | T | T | - | - |
| | ai | 0-1 | T | - | - |
| C14 | n | 2-4 | 2-3 | T | T |
| | i | 2-5 | T | T | T |
| C15 | n | T | 0-1 | - | - |
| | ai | 3-10 | 0-1 | T | T |
| C16:1 | | 0-1 | - | - | - |
| C16 | n | 4-5 | 62-71 | T | 2-19 |
| | i | 3-11 | 1-2 | 0-1 | 1-2 |
| C17 | n | T | 0-1 | - | T |
| | ai | 3-6 | 0-1 | 1-4 | T |
| C18 | n | 2-4 | 1-3 | 0-1 | 1-4 |
| | i | 4-7 | 8-13 | 0- 1 | 20-28 |
| C18:1 | | 0-1 | - | - | - |
| C19 | n | T | 0-1 | - | T |
| | ai | 4-7 | T | 1-3 | 2-5 |
| C20 | n | 0-1 | T | 1-3 | 1-5 |
| | i | 4-7 | 0-1 | 7-18 | 9-12 |
| C21 | n | T | T | T | T |
| C21 | ai | 4-6 | T | 9.25 | 5-10 |
| C22 | n | 0-1 | T | 1-3 | 1-2 |
| | i | 2-3 | 0-1 | 3-6 | 12-22 |
| C23 | n | T | T | T | T |
| | ai | 2-3 | 1-2 | 2-4 | 4-14 |
| C24 | n | 2-6 | T | 2-9 | 0-1 |
| | i | 2-3 | 1-2 | 3-5 | 3-12 |
| C25 | n | T | T | 0-1 | T |
| | ai | 5-6 | 0-1 | 6-8 | 1-2 |
| C26 | n | 2-5 | T | 1-7 | - |
| | i | 3-6 | -T | 6-13 | 0-1 |
| C27 | n | T | T | T | - |
| | ai | 3-6 | T | 9-16 | T |
| 28 | n | 1-2 | T | 0-2 | - |
| | i | 2-3 | T | 1-7 | T |
| C29 | n | T | T | - | - |
| | ai | 1-4 | T | 2-4 | T |
| C30 | n | 0-1 | T | T | - |
| | i | 1-3 | T | 1-3 | T |
| C31 | n | T | T | - | |
| | ai | 1-4 | - | 0-1 | - |
| C32 | n | T | T | T | |
| | i | 0-2 | - | 0-1 | - |
| C33 | n | - | - | - | - |
| | ai | 0-2 | - | T | - |

### • Spécifications de la lanoline :

La lanoline est une cire, solide de couleur jaune ambrée et d'odeur caractéristique. Elle est insoluble dans l'eau mais soluble dans l'éthanol à chaud, l'éther et le chloroforme.

Le tableau suivant rassemble les caractéristiques physico-chimiques de ce produit :

**Tableau n°3 : spécifications de la lanoline**

| **Caractéristiques** | **Lanoline** |
|---|---|
| Densité à 25°C | 0.88-0.96 |
| Point de fusion °C | 36-40 |
| Indice de réfraction à 40°C | 1.480 |
| Indice d'acide | 1-4 |
| Indice de saponification | 91-118 |
| Indice d'iode | 20-38 |
| Indice d'hydroxyle | 100-144 |
| lnsaonifiables % | 44-53 |
| Hydrocarbures % | 0-1 |
| Acides libres % | 0-1 |
| Acides totaux % | 48-55 |

### • Obtention de la lanoline :

La lanoline est extraite du suint du mouton, matière sébacée secrétée par les glandes sudoripares de l'animal. Le suint représente 15 à 75% du poids des fibres de la laine.

La peau lainée du mouton tué est expédiée vers les industries de délainage. A l'issue de l'opération de pelage, la peau et la laine sont séparées.

La peau est séchée et donne le cuirot qui sera envoyé à la mégisserie.

La laine, de son côté, est encore graisseuse : tout le suint se trouve sur le poil. Pour l'éliminer, la laine est trempée dans un bain de soude et de savon. Elle est alors prête aux opérations de cardage (brossage, démêlage) et de peignage (élimination des fibres courtes). Le suint est réutilisé, après raffinage, en pharmacie et cosmétologie.

### • Principaux producteurs de laine :

De nos jours, le commerce de la laine s'établit principalement entre les pays exportateurs de laine brute (Australie et Nouvelle-Zélande surtout) et les pays gros producteurs de filés de laine comme les Etats-Unis, l'Italie, le Japon et la Corée. Seuls l'Ex-URSS et la Chine produisent à la fois la laine brute et les filés.

### • Baisse considérable de la consommation des fibres naturelles comme la laine :

La laine est utilisée, depuis le néolithique, pour la confection des textiles. Néanmoins, la demande constante de produits lainiers est en partie satisfaite par des produits synthétiques dont les qualités concurrencent celles des draps et des tricots, héritiers d'une longue tradition. La part des fibres artificielles ne cesse d'augmenter partout dans le monde (fibres de cellulose issues du bois et de déchets de coton, les polyamides, les polyacryliques et les polyesters, issus du pétrole). En 1990, l'industrie des fibres chimiques a couvert près de 45% de la consommation mondiale de fibres textiles avec 42.5 millions de tonnes.

### - Fonctions et applications de la lanoline :

L'emploi de la lanoline et de ses dérivés (acétylée, éthoxylée, hydrogénée, hydroxylée...) est très fréquent en cosmétique et dans le secteur industriel.

La lanoline fut, dans un premier temps, utilisée dans le secteur pharmaceutique dans des pommades pour stabiliser les principes actifs dissous dans l'eau. Puis, elle devient très prônée dans des formulations pour ses propriétés émolliente et hydratante.

### - fonctions et applications cosmétiques et dermatologiques

La lanoline est très utilisée en cosmétique et dermatologie pour ses propriétés émolliente, hydratante, émulsionnante, occlusive. Elle assouplit la peau, la protège et empêche sa déshydratation. La lanoline a le pouvoir également d'absorber jusqu'à 4 fois son poids en eau. De plus, elle est peu sensible à l'oxydation.

Les dérivés de lanoline, extrêmement nombreux, obtenus par transformation physique ou chimique, présentant de meilleures caractéristiques d'odeur, de couleur et de toucher, sont préférentiellement utilisés.

De par ses propriétés remarquables, la lanoline et ses dérivés entrent dans la composition de nombreuses préparations comme : les bains moussants, les rouges à lèvres, les crèmes, les crèmes à raser, lotions après rasage, poudres, savons, shampooings.

### - fonctions et applications industrielles

La lanoline est également utilisée dans le secteur industriel avec diverses applications : cirages, encres d'imprimerie, pellicules protectrices antirouille et anticorrosion déposées sur certains métaux, industrie du cuir et de la fourrure pour l'imperméabilisation, les peintures...

Les raisons pour substituer la lanoline

Malgré toutes ses propriétés, la lanoline est de plus en plus critiquée pour les raisons suivantes :

### - son origine animale.

Les consommateurs d'aujourd'hui sont de plus en plus soucieux de préserver leur santé et la nature. Ils apprécient de plus en plus la présence d'ingrédients d'origine végétale dans les produits qu'ils utilisent. Les nombreuses enquêtes entreprises sur la tremblante du mouton (ESST : encéphalopathie spongiforme subaiguë transmissible) et la maladie de la vache folle (ESB encéphalopathie spongiforme bovine) inquiètent les consommateurs qui craignent d'être contaminés et d'attraper une variante de la maladie de Creutzfeldt-Jakob (MCJ). La tremblante du mouton est connue depuis la moitié du XVIII ème siècle et ce n'est qu'en 1985 que l'équivalent de cette maladie des ovins a été décrite chez les vaches. Les enquêtes ont montré que ces vaches avaient été nourries avec des farines animales, produites à partir de carcasses de moutons atteints de la tremblante et sans être chauffées à hautes températures. Une maladie spécifique du mouton s'est donc transmise à la vache qui elle-même peut contaminer l'homme par MCJ.

### - ses éventuelles impuretés et les réactions allergiques

La laine renferme naturellement des impuretés comme : l'herbe, la terre, les graterons et également des substances contaminantes : les pesticides. Même si la laine est nettoyée de ces impuretés avant d'être dégraissée, les traces de pesticides dans la lanoline (suint) ne sont pas exclues, ce qui freine les consommateurs, soucieux de la qualité des produits et de leur santé. Certains sujets ont montré des réactions allergiques à des produits cosmétiques contenant de la lanoline, non exempte de résidus pesticides et d'alcools polycycliques.

### - sa forte odeur

La lanoline possède une odeur forte caractéristique qui nécessite l'emploi d'une grande quantité d'agents parfumants pour masquer cette dernière. Cet ajout important de fragrances n'est pas toujours bien toléré chez les personnes aux peaux sensibles.

### - sa composition variable

La composition de la lanoline est complexe et variable (voir tableau n°1). Cette variabilité s'explique entre autres par la diversité des espèces des moutons, leurs âges, leurs types d'alimentation, le lieu. Aussi, il s'avère difficile d'obtenir de la lanoline de qualité constante.

Pour toutes ses raisons, de nombreux scientifiques cherchent à substituer la lanoline.

### Les alternatives de substitution de la lanoline

La première solution envisagée consiste à recréer la formule de la lanoline en excluant des ingrédients d'origine animale. La complexité de la composition de la lanoline (voir tableaux n°1 et 2) rend cette solution difficile : les coûts occasionnés sont trop onéreux pour continuer sur cette voie.

Une deuxième solution pour pallier ce problème consiste à fabriquer un produit de composition différente à celle de la lanoline mais aux propriétés semblables.

Jusqu'à présent, les substituts proposés n'apportent pas entière satisfaction : ils sont émollients, hydratants mais ils n'ont pas le pouvoir d'absorber l'eau.

De plus, la complexité de leur composition, certaines caractéristiques physico-chimiques élevées comme l'indice d'acide, et/ou leur coût de fabrication demandent à être optimisés.

Il existe donc un besoin important et reconnu de trouver un réel substitut de la lanoline.

### Résumé de l'invention :

La présente invention propose un produit industriel nouveau en soi, qui forme un substitut de lanoline, et est caractérisé en ce qu'il est capable d'absorber jusqu'à au moins deux fois son poids en eau ou en solution aqueuse ou en suspension aqueuse. Il forme de réelles émulsions, stables dans le temps avec un pouvoir de rétention d'eau supérieur à celui de la lanoline.

Ce substitut de lanoline est obtenu par hémi-synthèse à partir d'un mélange d'esters d'acides gras insaturés, et de composés comportant ou générant des fonctions hydroxyles, de préférence sans catalyseur et dans une atmosphère dépourvue d'oxygène.

### Applications :

Le Demandeur a découvert un substitut de lanoline pour le marché cosmétique et dermatologique et toutes les autres applications de la lanoline, et un procédé de préparation original.

Le Demandeur a découvert un substitut de lanoline pour le marché cosmétique et de par sa capacité d'absorption en eau ou solution aqueuse ou suspension aqueuse et ses propriétés émolliente, hydratante, il peut entrer dans la composition de nombreuses préparations.

### Exemples d'applications cosmétiques :

- les bains moussants,
- les rouges à lèvres,
- les crèmes de soin pour peaux sèches,
- les crèmes à raser,
- les lotions après rasage,
- poudres,
- savons,
- shampooings,
- les crèmes solaires waterproof,
- les démaquillants,
- les huiles pour le bain,
- les baumes à lèvres
- les onguents
- les sticks à lèvres...

### Exemples d'applications industrielles :

- cirages
- encres d'imprimerie,
- pellicules protectrices antirouille et anticorrosion déposées sur certains métaux,
- industrie du cuir et de la fourrure pour l'imperméabilisation,
- additifs pour lubrifiants,
- les peintures...

Cette liste n'est pas exhaustive. Ce substitut de lanoline est utilisable dans toutes les applications actuelles et futures de la lanoline.

L'invention concerne en particulier le procédé selon la revendication 1, de préférence sans catalyseur et dans une atmosphère dépourvue d'oxygène.

### Description de l'invention

La présente invention propose un produit industriel nouveau en soi, qui est un substitut de lanoline, et est caractérisé en ce qu'il est capable d'absorber jusqu'à au moins deux fois son poids en eau ou en solution aqueuse ou en suspension aqueuse. Il forme de réelles émulsions stables dans le temps avec un pouvoir de rétention d'eau supérieur à celui de la lanoline.

Il possède également les propriétés émollientes, hydratantes et occlusives connues de la lanoline. Il assouplit la peau, la protège et empêche sa déshydratation.

Son procédé de fabrication est simple et économique par rapport aux précédents.

Ce substitut de lanoline est obtenu par hémi-synthèse à partir d'un mélange d'esters d'acides gras insaturés, et de composés comportant ou générant des fonctions hydroxyles, de préférence sans catalyseur et dans une atmosphère dépourvue d'oxygène.

L'invention s'attache aux propriétés de ce produit.

De manière surprenante, il a le pouvoir d'absorber jusqu'à au moins 2 fois son poids en eau. De plus, les émulsions formées sont stables dans le temps et après plusieurs passages en étuve à 40 C. Ce phénomène n'est pas observé pour la lanoline qui au contraire relargue toute son eau en moins de 15 jours.

Ajouté à cette capacité d'absorption en eau, le produit est émollient, hydratant, émulsionnant et occlusif. Il assouplit la peau, la protège et empêche sa déshydratation.

La particularité de cette invention vient de l'ajout de réactifs tels que revendiqués comportant ou générant des fonctions hydroxyles dans le milieu réactionnel. Par simplicité, ces réactifs sont nommés réactifs-OH.

### La base polymérique et ses propriétés

Le Demandeur a déposé une demande de brevet FR 98 13770 et une demande de brevet PCT WO 00/26265 (PCT/FR99/02646) concernant un procédé original de chauffage diélectrique. Ce procédé s'applique particulièrement à la préparation du substitut de lanoline envisagé ici.

Selon ces brevets, le procédé de polymérisation d'acides gras insaturés, d'esters d'acides gras insaturés, d'hydrocarbures insaturés, de dérivés insaturés de ces composés, seuls ou en mélange, est caractérisé par le fait que le réactif ou le mélange réactionnel est soumis à un chauffage diélectrique pour opérer la polymérisation.

De préférence :
- Le chauffage est effectué par utilisation de fréquences micro-ondes.

Le chauffage est effectué par utilisation de fréquences radio.
- Il est mis en œuvre avec ou sans catalyseurs.
- On peut ajouter au réactif ou au mélange réactionnel des catalyseurs hétérogènes ou homogènes.
- On peut ajouter au réactif ou au mélange réactionnel des catalyseurs répondant aux fréquences radio ou aux fréquences micro-ondes, tel que la montmorillonite.
- Le réactif ou le mélange réactionnel, et éventuellement le (s) catalyseur (s), est placé dans un réacteur de type batch ou discontinu adapté pour recevoir des fréquences micro-ondes ou fréquences radio.
- Le réactif ou le mélange réactionnel, et éventuellement le (s) catalyseur (s), est placé dans un réacteur adapté pour faire des réactions en continu.
- Les fréquences sont comprises entre environ 30 GHz et environ 300 MHz.
- Les fréquences sont 2,45 GHz ou 915 MHz.
- Les fréquences sont comprises entre environ 300 MHz et environ 3 MHz.
- Les fréquences sont 13,56 MHz ou 27,12 MHz.
- La température à laquelle est soumis le réactif ou le mélange réactionnel, et éventuellement le (s) catalyseur (s), est comprise entre 200 et 400 °C, préférentiellement entre 220 et 350 °C.
- Le temps de montée en température est choisi entre 3 et 60 minutes, de préférence entre 3 et 20 minutes.
- Le temps de réaction est compris entre 15 minutes et 15 heures, de préférence entre 15 minutes et 360 minutes, de préférence encore entre 15 et 120 minutes.
- On effectue la polymérisation sous atmosphère normale ou riche en oxygène ou de préférence inerte ; sous pression réduite, de préférence entre 50 et 10 mm de mercure ; en renouvelant régulièrement l'atmosphère.
- On arrête la polymérisation en laissant refroidir ou en refroidissant le réactif ou le mélange réactionnel à une température inférieure à la température de polymérisation et ce suivant la viscosité que l'on souhaite obtenir.

Cependant, on pourra aussi opérer selon un traitement thermique classique.

Sans l'ajout de ces « réactifs OH », le Demandeur obtient avec le procédé énoncé ci-dessus des polymères d'acides gras insaturés, d'esters d'acides gras insaturés, d'hydrocarbures insaturés ou de dérivés de ces produits.

Ces polymères possèdent à eux seuls des propriétés émolliente, hydratante et occlusive, prônées pour les formulations cosmétiques. Ils ont également le pouvoir d'absorber l'eau mais ce pouvoir reste limité, comparé à celui de la lanoline : ils absorbent jusqu'à 80% de leur poids en eau. La structure du réseau polymérique formé suffit pour emmagasiner de l'eau et former des émulsions. Néanmoins, ces émulsions ne sont pas stables : l'eau est relarguée en moins de 24 heures.

Un problème technique a été de faire en sorte que les propriétés des polymères obtenus se rapprochent le plus près possible de celles de la lanoline. Pour cette raison, on ajoute dans le milieu réactionnel une quantité suffisante de réactifs-OH c'est-à-dire comportant ou générant des groupements hydroxyles.

On a observé de manière surprenante que les produits obtenus conservent les propriétés initiales (émolliente, hydratante, occlusive) mais absorbent maintenant jusqu'à au moins deux fois leur poids en eau. De plus, les émulsions formées sont stables dans le temps et après plusieurs passages en étuve à 40 °C. Ce phénomène n'est pas observé pour la lanoline qui relargue toute son eau en moins de 15 jours.

Ces produits ont un pouvoir de rétention d'eau supérieur à celui de la lanoline.

Il est à noter que l'ajout de réactifs-OH comportant ou générant des groupements hydroxyles permet également de réduire l'acidité des produits formés par estérification et/ou amidification, limitant ainsi les risques de dégradation thermique et les réactions d'hydrolyse. Les produits formés sont ainsi beaucoup plus stables.

De même, l'ajout de réactifs-OH dans un mélange initial donné d'esters d'acides gras insaturés, modifie la viscosité du produit final.

### La fabrication de ces substituts de lanoline

Le substitut de lanoline est obtenu selon le procédé revendiqué, de préférence sans catalyseur et dans une atmosphère dépourvue d'oxygène.

Le traitement thermique est effectué en soumettant les réactifs à un chauffage classique ou diélectrique comme décrit dans les brevets précités du Demandeur.

### A Traitement thermique classique :

Le traitement thermique classique est effectué entre 100 et 400 °C et mieux encore entre 230 et 350 °C, sous agitation permanente et préférentiellement sans catalyseur et sous atmosphère inerte. La température réactionnelle dépend des températures d'ébullition et/ou de dégradation des constituants du mélange.

Le temps total de réaction dépend du ou des réactifs utilisés et de la viscosité que l'on souhaite obtenir. Il se situe de préférence entre 3 heures et 24 heures, de préférence entre 3 heures et 10 heures.

### B Traitement par chauffage diélectrique :

Pour des raisons de gains de temps et d'énergie, combinés à un coût d'investissement plus faible, on utilisera préférentiellement le chauffage diélectrique, c'est à dire un chauffage sous fréquences micro-ondes ou hautes fréquences, comme décrit dans les brevets précités, dont on a donné ci-dessus un extrait pour la convenance de l'homme de métier, et auquel celui-ci pourra se reporter utilement pour les détails de mise en œuvre. L'homme de métier pourra également se reporter à la demande de brevet déposée ce même jour par le Demandeur, et concernant un appareillage perfectionné pour la mise en œuvre du chauffage diélectrique.

On opère de préférence sans catalyseur, et il est tout-à-fait surprenant de constater que l'on parvient, sans catalyseur, à une estérification des acides gras libres par un produit réactif comme le glycérol.

Il est également très surprenant de constater qu'il ne se développe pas de réactions parasites ou concurrentes, comme cela était au contraire prévisible, ce qui a peut-être bloqué les recherches dans cette voie, et que l'on obtient donc un bon rendement.

Les fréquences micro-ondes MO sont comprises entre environ 300 MHz et environ 30 GHz, préférentiellement à 915 MHz (fréquence autorisée avec une tolérance de 1.4%) ou à 2.45 GHz (fréquence autorisée avec une tolérance de 2%).

Les hautes fréquences HF sont comprises entre environ 3 MHz et environ 300MHz, préférentiellement à 13.56 MHz (fréquence autorisée avec une tolérance de 0.05%) ou à 27.12 MHz (fréquence autorisée avec une tolérance de 0.6%).

Les températures de réaction se situent entre 100 et 400°C et mieux encore entre 230 et 350 °C, sous agitation permanente et préférentiellement sans catalyseur et sous atmosphère inerte. La température réactionnelle dépend des températures d'ébullition et/ou de dégradation des constituants du mélange.

Le temps total de réaction dépend du ou des réactifs utilisés et de la viscosité que l'on souhaite obtenir. Il se situe de préférence entre 15 minutes et 15 heures, de préférence entre 15 minutes et 2 heures.

La quantité des réactifs-OH ajoutée au milieu réactionnel dépend du niveau d'absorption en eau que l'on souhaite atteindre pour le polymère résultant ainsi que de la viscosité attendue. Les réactifs-OH peuvent être introduits dans le milieu réactionnel en début, en cours ou en fin de réaction.

L'ajout de ces produits en fin de réaction n'est généralement pas avantageux économiquement car les temps de réaction sont augmentés. On peut cependant y avoir recours dans certains cas.

Les produits obtenus par ce procédé peuvent subir des traitements supplémentaires tels que l'hydrogénation, la décoloration, la désodorisation ou autres fonctionnalisations si ces traitements apportent des propriétés supplémentaires comme l'odeur, la couleur...

### Les réactifs (mode traitement thermique classique ou par chauffage diélectrique)

Pour la présente invention, les réactifs peuvent être choisis parmi les huiles et graisses végétales et certains polyterpènes peuvent être issus desdites huiles et graisses.

En tant qu'huiles d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, huile de carthame, huile de noyaux d'abricot, l'huile d'amande douce, l'huile de chanvre, l'huile de pépins de raisin, l'huile de coprah, l'huile de palme, l'huile de graine de coton, l'huile de babassu, l'huile de jojoba, l'huile de sésame, l'huile d'argan, l'huile de chardon marie, huile de pépins de courge, huile de framboise, l'huile de Karanja, l'huile de Neem, l'huile d'oeillette, l'huile de noix du Brésil, l'huile de ricin, l'huile de ricin déshydratée, l'huile de noisette, l'huile de germe de blé, l'huile de bourrache, l'huile d'onagre, l'huile de Tung, l'huile de tall ou « tall oil ».

Ces huiles et graisses d'origine végétale, ainsi que leurs dérivés, peuvent subir un traitement préalable visant à les rendre plus réactifs ou au contraire moins réactifs.

L'invention concerne aussi bien un réactif isolé qu'un mélange réactionnel comportant deux ou plusieurs composants. Ces mélanges réactionnels peuvent comporter des proportions équivalentes de chaque composant ou certains composants peuvent être majoritaires.

En tant qu'esters d'acides gras insaturés, on peut utiliser seul ou en mélange et à titre d'exemples non limitatifs, un ou des esters obtenus par estérification entre un monoalcool et/ou polyol et au moins un acide gras insaturé ; des cires ; des phospholipides ; des sphingolipides ; des glucolipides. Les huiles et graisses d'origine végétale, peuvent subir un traitement préalable visant à les rendre plus réactifs ou au contraire moins réactifs comme par exemple l'hydrogénation, l'hydroxylation, l'époxydation, la phosphitation, la sulfonation.

Bien que les huiles et les graisses d'origine animale ne soient pas préférentiellement utilisées, on peut citer entre autres, l'huile de cachalot, l'huile de dauphin, l'huile de baleine, l'huile de phoque, l'huile de sardine, l'huile de hareng, l'huile de squale, l'huile de foie de morue, l'huile de pied de boeuf, les graisses de porc, de cheval.

En tant qu'alcools, on utilise un ou plusieurs composés choisis parmi le glycérol, le polyglycérol, le sorbitol, la monoéthanolamine MEA, et les vitamines C et E.

Les alcools, les aminoalcools, ainsi que leurs dérivés peuvent subir un traitement préalable visant à les rendre plus réactifs ou au contraire moins réactifs comme par exemple l'hydrogénation, l'hydroxylation, l'époxydation, la phosphitation, la sulfonation.

Parmi les catalyseurs ou adjuvants, on entendra à titre d'exemples non limitatifs les catalyseurs acides usuels (acide paratoluènesulfonique, acide sulfurique, acide phosphorique, acide perchlorique...), les catalyseurs basiques usuels (soude, potasse, alcoolate de métaux alcalins et de métaux alcalino-terreux, acétate de sodium, triéthylamines, dérivés de pyridine...), les résines acides et/ou basiques de type AmberliteTM, AmberlysFM, PuroliteTM, DowexTM, LewatiFM, les zéolithes et les enzymes, les noirs de carbone et les fibres de carbone activées.

L'invention concerne un procédé caractérisé en ce que on ajoute de 0,1 à 30%, de préférence de 0,1 à 6 % de « réactif-OH », de préférence 1 ou 3 %, notamment de 0,1 à 5, de préférence 1 ou 3 % de glycérol et le réactif OH est choisi parmi le glycérol, le sorbitol, la MEA, le polyglycérol, la vitamine C ou E.

Des exemples concrets mais non limitatifs de l'invention vont être maintenant présentés.

### EXEMPLES

Les exemples suivants permettent de mettre en évidence le fait que la capacité d'absorption en eau de la base polymérique, formée à partir d'un mélange d'esters d'acides gras insaturés est augmentée en ajoutant au milieu réactionnel des composés comportant ou générant des fonctions hydroxyles.

### 1- ABSORPTION EN EAU

### 1-Protocoles de mise en émulsion

Les essais de capacité d'absorption en eau sont réalisés de deux manières :
1-mise en émulsion en chauffant à 60 °C à l'aide d'un barreau aimanté (protocole n 1)
2-mise en émulsion en chauffant à 60 °C à l'aide d'une pale à turbine (protocole n 2)

### a- protocole n°1

### Matériel nécessaire :

- béchers de 250mL
- agitateur magnétique à plaque chauffante
- burette de 50mL

### Protocole :

- peser une masse m1 du substitut de lanoline dans un bécher
- chauffer le produit à 60°C sous agitation (barreau aimanté) à l'aide d'un appareil chauffant
- ajouter goutte à goutte à l'aide d'une burette graduée l'eau froide jusqu'à ce que le produit ne l'absorbe plus
- noter la masse m2 de l'eau absorbée
- évaluer la capacité d'absorption (m2/m1*100)
- contrôler périodiquement la stabilité de l'émulsion

Ce protocole permet d'avoir une bonne idée de l'efficacité des produits.

### b- protocole n°2

Le protocole n 2 est plus précis et plus complet que le protocole n01.

### Matériel nécessaire :

- béchers inox (100-250-500mL)
- bain marie
- agitateur HEIDOLPH, 280-2200t/min (échelle de 0 à 10) avec turbine défloculeuse
- pipettes
- spatule
- pHmètre METTLER TOLEDO MP220
- centrifugeuse HETTICH UNIVERSAL (4000U/min à 100%)

### Réactifs nécessaires :

- solution aqueuse bleue 1 à 0. 1%
- solution Kathon CG
- solution pH= 7 et 4 et 11

### Protocole :

1-peser l'huile dans un bécherinox 500mL (m1)
2-chauffer l'huile dans un bain marie à 60 °C
3-agiter à 4
4-peser l'eau (m2, ici 200%) dans un bécher inox 250mL
5-ajouter dans l'eau 3 gouttes de solution bleue et 3 gouttes de Kathon CG
6-chauffer l'eau dans un autre bain marie à 80 °C (thermomètre)
7-ajouter goutte à goutte l'eau à l'aide d'une pipette
8-une fois l'émulsion prise, ajouter rapidement le reste d'eau en agitant à 6
9-attendre 5 minutes après ajout pour que l'émulsion soit terminée
10- refroidir l'émulsion dans un bain d'eau froide en remuant avec la spatule
11- vérifier la conductivité de l'émulsion au pHmètre étalonné
12-centrifuger pendant 15min à 4000U/min (100%)
13-une fois terminé, observer ou non le déphasage (mis en évidence par la solution bleue)
14-effectuer des tests de stabilité dans le temps par passage à l'étuve à 40 et 50 °C.

### c-Légende :

CAE (1) = capacité d'absorption en eau obtenue en suivant le protocole n°1, exprimée en %
CAE (2) = capacité d'absorption en eau obtenue en suivant le protocole n°2, exprimée en %
S (1) = stabilité de l'émulsion obtenue en suivant le protocole n°1, exprimée en jours
S (2) = stabilité de l'émulsion obtenue en suivant le protocole n 2, exprimée en jours
R (2) = relargage de l'eau après passage à la centrifugeuse, exprimé par oui/non

### 2-Influence de l'ajout de glycérol et de la viscosité sur la capacité d'absorption en eau

les produits qui figurent dans les tableaux ci-dessous sont obtenus à partir du traitement thermique des mêmes esters d'acides gras insatures.

Seules les proportions de chaque composé dans le mélange varient, de manière à obtenir en fin de synthèse la viscosité voulue.

**Tableau n°5 :la lanoline**

| **Référence** | **CAE (2)** | **S(2) à 25°C** |
|---|---|---|
| Lanoline | >200 | stable |

L'objectif est d'atteindre ces deux critères : une capacité d'absorption en eau supérieure à 200% et une stabilité dans le temps de l'émulsion formée.

**Tableau n°6 : Viscosité des bases polymériques sans glycérol**

| **0% GLYCEROL** | **VISCOSITE cP (40°C)** | **CAE (1)** | **S(1) à 25°C** |
|---|---|---|---|
| CM137/00 | 600 | 80 | <1 jour |
| CM34/01 | 1800 | 70 | <1 jour |
| CM120/00, CM121/00 | 2500 | 80 | <1 jour |
| CM113/00, CM87/00 | 4500 | 80 | <1 jour |
| CM91/00 | 10 000 | 42 | <1 jour |

Sans l'ajout de réactifs-OH, les polymères d'esters d'acides gras insaturés absorbent jusqu'à 80% de leur poids en eau.

La structure du réseau polymérique formée permet d'emmagasiner l'eau. Le pouvoir d'absorption en eau reste cependant limité, comparé à celui de la lanoline. De plus, l'eau est relarguée en moins d'un jour.

Il est également important de noter que l'eau pénètre difficilement dans des réseaux polymériques complexes (exemple : CM91/00).

**Tableau n 7 : Viscosités des substituts de lanoline obtenus avec 3% de glycérol**

| **3% GLYCEROL** | **VISCOSITE cP 40°C** | **CAE (1)** | **CAE(2)** | **R(2)** | **S(1) 25°C** | **S(2) 25°C** |
|---|---|---|---|---|---|---|
| CM76/01) | 600 | 170 | >200 | non | 15 jours | 15 jours |
| CM78/01 | 1000 | 280 | >200 | non | 30 jours | stable |
| CM80/01 | 1800 | 280 | >200 | non | stable | stable |
| CM81/01 | 2500 | 295 | >200 | non | stable | stable |
| CM82/01 | 4500 | 200 | >200 | non | stable | stable |
| CM99/01 | 10 000 | 200 | >200 | non | stable | stable |

Le glycérol est ajouté dans le mélange initial, avant de commencer la synthèse.

Les produits obtenus absorbent alors jusqu'à au moins deux fois leur poids en eau.

Les émulsions formées sont stables à température ambiante sauf pour les produits peu visqueux (600 et 1000 cP à 40°C). Pour que ces derniers soient stables, il faut réduire la quantité d'eau absorbée.

Leur capacité d'absorption en eau se situe entre 80 et 200%.

Le choix du produit et de sa viscosité dépend de l'application et de l'effet souhaités. Plus le produit est visqueux, plus l'émulsion obtenue est épaisse.

### 3- Stabilité des émulsions

Les émulsions formées subissent différents cycles « étuve à 40°C-centrifugeuse » pour contrôler leur stabilité.

Les résultats obtenus figurent dans le tableau ci-dessous :

**Tableau n°8 : test de stabilité à l'étuve à 40 °C**

| **3% GLYCEROL** | **VISCOSITÉ cP 40°C** | **CAE(2)** | **S(2) 25°C** | **S(2) CYCLES** |
|---|---|---|---|---|
| CM76/01 | 600 | >200 | 15 jours | 15 jours |
| CM78/01 | 1000 | >200 | stable | stable |
| CM80/01 | 1800 | >200 | stable | stable |
| CM81/01 | 2500 | >200 | stable | stable |
| CM82/01 | 4500 | >200 | stable | stable |
| CM99/01 | 10 000 | >200 | stable | stable |
| Lanoline | - | >200 | stable | 15 jours |

Les produits de viscosité supérieure à 1000cP (à 40°C) absorbent plus de deux fois leur poids en eau et forment de réelles émulsions.

Ces émulsions sont stables contrairement à la lanoline qui relargue la totalité de son eau en 15 jours après passage en étuve à 40°C.

Ces substituts de lanoline ont donc un pouvoir d'absorption en eau équivalent à celui de la lanoline mais son pouvoir de rétention est meilleur.

### 4 Influence du pourcentage de glycérol

Les produits qui figurent dans le tableau ci-dessous, sont obtenus à partir du traitement thermique du même mélange d'esters d'acides gras insaturés (nature des produits et proportions identiques).

Seule la quantité de glycérol introduite dans le mélange varie.

**Tableau n 9 : capacité d'absorption en eau fonction du pourcentage de glycérol ajouté**

| **NOM PRODUIT** | **% GLYCEROL** | **VISCOSITE cP 40°C** | **CAE (1)** | **S(1)** |
|---|---|---|---|---|
| CM20/01 | 0 | 900 | 80 | <1 jour |
| CM28/01 | 1 | 850 | 80 | <1 jour |
| CM38/01 | 3 | 500 | 170 | 15 jours |
| CM29/01 | 5 | 450 | 190 | 15 jours |

La capacité d'absorption en eau augmente avec le pourcentage de glycérol.

Les émulsions formées ne sont pas stables. Comme précédemment, les produits ne sont pas assez visqueux : la base polymérique n'apporte pas suffisamment de consistance.

Il est à noter que la viscosité d'un mélange donné diminue avec le pourcentage de glycérol.

### 5-Comparaison des capacités d'absorption de différents réactifs

Les produits qui figurent dans le tableau ci-dessous, sont obtenus à partir du traitement thermique du même mélange d'esters d'acides gras insaturés (nature des produits et proportions identiques).

Seul le composé comportant ou générant des fonctions hydroxyles change. La quantité à ajouter au milieu réactionnel varie en fonction des fonctions hydroxyles du réactif-OH qui peuvent réagir au cours de la synthèse et celles qui ne réagissent pas pour des raisons d'encombrement stérique.

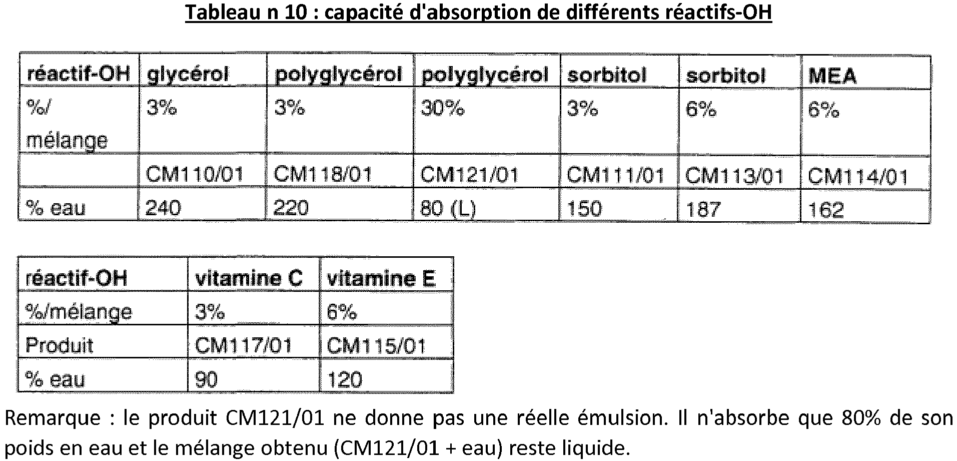

### II- FORMULATIONS

### 1- Propriétés émolliente, hydratante et occlusive d'une huile de tournesol polymérisée sans OH

Le Demandeur a déposé une demande de brevet FR 98 13770 et une demande de brevet PCT WO 00/26265 (PCT/FR99/02646).

Dans ce brevet, il montre les avantages d'une crème de soin pour les mains formulée en remplaçant tout ou partie des ingrédients par une huile de tournesol polymérisée (HTP)

**Tableau n 11 : Formule d'une crème de soin pour les mains**

| | | **Formule initiale** | **Formule modifiée** |
|---|---|---|---|
| **COMPOSANT** | **FONCTION** | % | % |
| **HTP** | **Substitut** | **0** | **5** |
| Alcool stéarique | Emulsionnant | 5 | 4 |
| Acide stéarique | Emulsionnant | 0,4 | 0,2 |
| Beurre de karité | Emollient/Emulsioonant | 1 | 0 |
| Eau | 66,6 | 66,6 | 73.8 |
| Glycérine | Hydratant « émulsionnant » | 20 | 10 |
| Autres ingrédients | Additifs divers | 7 | 7 |

Les bénéfices de la formule modifiée sont les suivants :
- Elle savonne moins à l'application ;
- Elle est plus émolliente (elle assouplit la peau) ;
- Elle forme une barrière qui préserve l'hydratation de la peau.

L'huile de tournesol polymérisée est avantageuse pour le formulateur de produits cosmétiques car :
- Le pouvoir émollient de la formule est supérieur alors que l'on a augmenté la proportion en eau ;
- L'HTP a un pouvoir viscosant ;
- L'HTP est un coémulsionnant (épaississant)

### 2-Applications avec émulsions

Le tableau ci-dessous décrit la formule d'une crème nutritive multifonctionnelle :

**Tableau n 12 : Formule d'une crème nutritive multifonctionnelle**

| **Ingrédients** | **Témoin** | **CM68/00** | **CM72/00** | **CM81/00** |
|---|---|---|---|---|
| Eau déminéralisée | QSP 100 | QSP 1 00 | QSP 100 | QSP 100 |
| Glycérine | 15 | 15 | 15 | 15 |
| Triéthanolamrne | 0.67 | 0.67 | 0,67 | 0.67 |
| Vaseline | 8 | 8 | 8 | 8 |
| Gtyceryf Stearate | 6 | 6 | 6 | 6 |
| Acide stéarique | 3 | 3 | 3 | 3 |
| Huile de vaseline | 2 | 2 | 2 | 2 |
| Diméthicone | 0.85 | 0.85 | 0.85 | 0.85 |
| Gyclomethicone | 2.5 | 2.5 | 2.5 | 2.5 |
| Conservateur | 0.5 | 0.5 | 0.5 | 0.5 |
| Acetate de vitamine E | 0.05 | 0.05 | 0.05 | 0.05 |
| Palmitate de vitamine A | 0.1 | 0.1 | 0.1 | 0.1 |
| Carbomer | 0.24 | 0.24 | 0.24 | 0.24 |
| **Alcools de lanoline** | **2** | **0** | **0** | **0** |
| **CM68/00 (600cP à 40°C)** | **0** | **2** | **0** | **0** |
| **CM72/00 (600cP à 40°C)** | **0** | **0** | **2** | **0** |
| **CM81/00 (2500cP à 40°C)** | **0** | **0** | **0** | **2** |

Les formulations à base des produits CM68/00 et CM72/00 apportent des propriétés cosmétiques intéressantes par rapport au témoin :
- pénétration rapide malgré un étalement légèrement moins bon.
- sensation finale de velours sur la peau.
- sensation huileuse diminuée.

### 3-Autres applications

### a- Onguent

| **Onguent témoin** | **Onguent Aldivia** | **Quantité (%)** |
|---|---|---|
| Vaseline | Vaseline | 50 |
| Lanoline | CM82/01 (v=4500cP, 3% glycérol) | 30 |
| Cire d'abeille | Cire d'abeitle | 5 |
| Huile de Neem | Huile de Neem | 5 |
| Beurre de Karité stabilisé | Beurre de Karité stabilisé | 10 |

### b-Stick à lèvres

| **Stick témoin** | **Quantité (%)** | **Stick Aldivia** | **Quantité (%)** |
|---|---|---|---|
| vaseline | 58.7 | Vaseline | 29.35 |
| | | CM82/01 ((v=4500cP, 3% glycérol) | 29.35 |
| Paraffine 52/54 | 10 | Paraffine 52/54 | 10 |
| Cerozo E626 | 10.5 | Cerozo 6626 | 10.5 |
| Cerewax A75 | 10.5 | Cerewax A75 | 10.5 |
| Crodamol ML | 10 | Crodamm ML | 10 |
| Acétate de vitamine E | 0.1 | Acétate de vitamine E | 0.1 |
| Propylparaben | 0.2 | Propylparaben | 0.2 |

### CONCLUSION

Ces exemples montrent l'efficacité de ces produits : ils constituent de réels substituts de la lanoline.

Ils ont le pouvoir d'absorber jusqu'à au moins 2 fois leur poids en eau et leur pouvoir de rétention est supérieur à celui de la lanoline.

Comme la lanoline, ils sont émollients, hydratants et occlusifs. Ils assouplissent la peau, la protègent et empêchent sa déshydratation.

Le choix du produit et de sa viscosité dépend de l'application et de l'effet souhaités. Plus le produit est visqueux et forme un réseau polymérique, capable d'emmagasiner l'eau, plus l'émulsion obtenue est épaisse et stable dans le temps.

La quantité de réactifs-OH ajouté au milieu dépend de la capacité d'absorption en eau souhaitée.

L'invention concerne également tous les modes de réalisation et variantes directement accessibles à l'homme de métier.

## Revendications

1. Procédé de polymérisation au moyen d'un traitement thermique d'esters d'acides gras insaturés, seuls ou en mélange avec des polyterpènes, **caractérisé en ce qu'**on ajoute dans le mélange réactionnel 1 à 6 % en poids du mélange, d'un ou plusieurs composés choisis parmi le glycérol, le polyglycérol, le sorbitol, la monoéthanolamine MEA, et les vitamines C et E.

2. Procédé selon la revendication 1, **caractérisé en ce que** les esters d'acides gras insaturés sont choisis parmi les huiles et graisses d'origine végétale.

3. Procédé selon la revendication 2, **caractérisé en ce que** les huiles d'origine végétale sont choisies parmi l'huile de colza, l'huile de tournesol, l'huile d'arachide, l'huile d'olive, l'huile de noix, l'huile de maïs, l'huile de soja, l'huile de lin, huile de carthame, huile de noyaux d'abricot, l'huile d'amande douce, l'huile de chanvre, l'huile de pépins de raisin, l'huile de coprah, l'huile de palme, l'huile de graine de coton, l'huile de babassu, l'huile de jojoba, l'huile de sésame, l'huile d'argan, l'huile de chardon marie, huile de pépins de courge, huile de framboise, l'huile de Karanja, l'huile de Neem, l'huile d'oeillette, l'huile de noix du Brésil, l'huile de ricin, l'huile de ricin déshydratée, l'huile de noisette, l'huile de germe de blé, l'huile de bourrache, l'huile d'onagre, l'huile de Tung, l'huile de tall ou « tall oil », qui peuvent subir un traitement préalable visant à les rendre plus réactives ou au contraire moins réactives.

4. Procédé selon la revendication 1, **caractérisé en ce que** en tant qu'esters d'acides gras insaturés, on utilise seul ou en mélange un ou des esters obtenus par estérification entre un monoalcool et ou polyol et au moins un acide gras insaturé ; des cires ; des phospholipides ; des sphingolipides ; des glucolipides.

5. Procédé selon la revendication 4, **caractérisé en ce que** les huiles et graisses d'origine végétale subissent un traitement préalable visant à les rendre plus réactives ou au contraire moins réactives, choisi parmi l'hydrogénation, l'hydroxylation, l'époxydation, la phosphitation, la sulfonation.

6. Procédé selon l'une quelconque des revendications 1 à 5, **caractérisé en ce que** le procédé est mis en œuvre sans catalyseur et dans une atmosphère dépourvue d'oxygène.

7. Procédé selon l'une quelconque des revendications 1 à 6, **caractérisé en ce que** le mélange réactionnel est soumis à un chauffage diélectrique pour opérer la polymérisation.

8. Procédé selon la revendication 7 **caractérisé en ce que** le chauffage est effectué par utilisation de fréquences micro-ondes.

9. Procédé selon la revendication 7 **caractérisé en ce que** le chauffage est effectué par utilisation de fréquences radio.

10. Procédé selon l'une quelconque des revendications 1 à 9, **caractérisé en ce qu'**on ajoute au réactif ou au mélange réactionnel des catalyseurs hétérogènes ou homogènes.

11. Procédé selon l'une quelconque des revendications 1 à 10, caractérisé en ce on ajoute au réactif ou au mélange réactionnel des catalyseurs répondant aux fréquences radio ou aux fréquences micro-ondes.

12. Procédé selon l'une quelconque des revendications 1 à 11, **caractérisé en ce que** le réactif ou le mélange réactionnel, et éventuellement le(s) catalyseur(s), est placé dans un réacteur de type batch ou discontinu adapté pour recevoir des fréquences micro-ondes ou fréquences radio.

13. Procédé selon l'une quelconque des revendications 1 à 12, **caractérisé en ce que** le réactif ou le mélange réactionnel, et éventuellement le(s) catalyseur(s), est placé dans un réacteur adapté pour faire des réactions en continu.

14. Procédé selon la revendication 8 ou 9, **caractérisé en ce que** les fréquences sont comprises entre environ 30 GHz et environ 300 MHz, entre 2,45 GHz ou 915 MHz, entre environ 300 MHz et environ 3 MHz et entre 13,56 MHz ou 27,12 MHz.

15. Procédé selon l'une quelconque des revendications 1 à 14, **caractérisé en ce que** la température à laquelle est soumis le réactif ou le mélange réactionnel, et éventuellement le(s) catalyseur(s), est comprise entre 200 et 400°C, préférentiellement entre 220 et 350°C.

16. Procédé selon la revendication 1 à 15, **caractérisé en ce que** le temps de montée en température est choisi entre 3 et 60 minutes, de préférence entre 3 et 20 minutes, lorsque le mélange réactionnel est soumis à un chauffage diélectrique.

17. Procédé selon l'une quelconque des revendications 1 à 16, **caractérisé en ce que** le temps de réaction est compris entre 15 minutes et 15 heures, de préférence entre 15 minutes et 360 minutes, de préférence encore entre 15 et 120 minutes.

18. Procédé selon l'une quelconque des revendications 1 à 17, **caractérisé en ce qu'**on effectue la polymérisation sous atmosphère riche en oxygène ou inerte ; sous pression réduite, de préférence entre 50 et 10 mm de mercure ; en renouvelant régulièrement l'atmosphère.

19. Procédé selon l'une quelconque des revendications 1 à 18, **caractérisé en ce qu'**on arrête la polymérisation en laissant refroidir ou en refroidissant le réactif ou le mélange réactionnel à une température inférieure à la température de polymérisation et ce suivant la viscosité que l'on souhaite obtenir.

20. Procédé selon l'une quelconque des revendications 1 à 19, **caractérisé en ce que** le réseau polymérique obtenu subit des traitements supplémentaires.

21. Procédé selon la revendication 20, **caractérisé en ce que** le traitement supplémentaire est choisi parmi l'hydrogénation, la décoloration et la désodorisation si ledit traitement thermique apporte des propriétés supplémentaires comme l'odeur, la couleur.

22. Réseau polymérique fabriqué selon un procédé tel que décrit à l'une quelconque des revendications 1 à 21.

23. Utilisation du réseau polymérique selon la revendication 22 pour la préparation d'une formulation cosmétique ou dermatologique.

24. Utilisation d'un réseau polymérique selon la revendication 22 comme émollient ou hydratant.

25. Utilisation d'un réseau polymérique selon la revendication 22 pour la préparation d'une formulation cosmétique ou dermatologique choisie parmi les bains moussants, les rouges à lèvres, les crèmes, les crèmes pour peaux sèches, les crèmes à raser, lotions après rasage, poudres, savons, shampooings, les crèmes solaires waterproof, les démaquillants, les huiles pour le bain, les baumes à lèvres, les onguents, les sticks à lèvres.

26. Utilisation d'un réseau polymérique selon la revendication 22 pour assouplir la peau, protéger la peau ou empêcher la déshydratation de la peau.

27. Utilisation d'un réseau polymérique selon la revendication 22 comme substitut de lanoline.

## Patentansprüche

1. Polymerisationsverfahren mittels einer thermischen Behandlung von ungesättigten Fettsäurenestern, allein oder gemischt mit Polyterpenen, **dadurch gekennzeichnet, dass** es dem Reaktionsgemisch 1 bis 6 Gew-.% des Gemischs von einer oder mehreren Verbindungen ausgewählt aus Glyzerin, Polyglyzerin, Sorbitol, Monoethanolamin MEA, und den Vitaminen C und E zugegeben wird.

2. Verfahren nach Anspruch 1, **dadurch gekennzeichnet, dass** die ungesättigten Fettsäurenester ausgewählt sind aus Ölen und Fetten pflanzlicher Herkunft.

3. Verfahren nach Anspruch 2, **dadurch gekennzeichnet, dass** die Öle pflanzlicher Herkunft ausgewählt sind aus Rapsöl, Sonnenblumenöl, Erdnussöl, Olivenöl, Walnussöl, Maisöl, Sojaöl, Leinöl, Distelöl, Aprikosenkernöl, Süßmandelöl, Hanföl, Traubenkernöl, Kokonußöl, Palmöl, Baumwollsamenöl, Babassuöl, Jojobaöl, Sesamöl, Arganöl, Mariendistelöl, Kürbiskernöl, Himbeeröl, Karanjaöl, Neemöl, Leinsamenöl, Paranussöl, Rizinusöl, dehydriertem Rizinusöl, Haselnussöl, Weizenkeimöl, Borretschöl, Nachtkerzenöl, Tungöl, Tallöl oder "tall oil", die einer Vorbehandlung unterzogen werden können, um diese reaktiver oder im Gegenteil weniger reaktiv zu machen.

4. Verfahren nach Anspruch 1, **dadurch gekennzeichnet, dass**, als ungesättigte Fettsäurenester, ein oder mehrere Ester, allein oder gemischt, eingesetzt werden, die durch Veresterung zwischen einem Monoalkohol und / oder Polyol und mindestens einer ungesättigten Fettsäure, Wachsen, Phospholipiden, Sphingolipiden, Glucolipiden erhalten werden.

5. Verfahren nach Anspruch 4, **dadurch gekennzeichnet, dass** die Öle und Fette pflanzlicher Herkunft einer Vorbehandlung unterzogen werden, um diese reaktiver oder im Gegenteil weniger reaktiv zu machen, ausgewählt aus Hydrierung, Hydroxylierung, Epoxidierung, Phosphitierung, Sulfonierung.

6. Verfahren nach irgendeinem der Ansprüche 1 bis 5, **dadurch gekennzeichnet, dass** das Verfahren ohne Katalysator und in einer sauerstofffreien Atmosphäre durchgeführt wird.

7. Verfahren nach irgendeinem der Ansprüche 1 bis 6, **dadurch gekennzeichnet, dass** das Reaktionsgemisch einem dielektrischen Erwärmen unterzogen wird, um die Polymerisation durchzuführen.

8. Verfahren nach Anspruch 7, **dadurch gekennzeichnet, dass** das Erwärmen unter Verwendung von Mikrowellenfrequenzen durchgeführt wird.

9. Verfahren nach Anspruch 7, **dadurch gekennzeichnet, dass** das Erwärmen unter Verwendung von Radiofrequenzen durchgeführt wird.

10. Verfahren nach irgendeinem der Ansprüche 1 bis 9, **dadurch gekennzeichnet, dass** dem Reagenz oder dem Reaktionsgemisch heterogene oder homogene Katalysatoren zugegeben werden.

11. Verfahren nach irgendeinem der Ansprüche 1 bis 10, **dadurch gekennzeichnet, dass** dem Reagenz oder dem Reaktionsgemisch Katalysatoren zugegeben werden, die auf Radiofrequenzen oder Mikrowellenfrequenzen reagieren.

12. Verfahren nach irgendeinem der Ansprüche 1 bis 11, **dadurch gekennzeichnet, dass** das Reagenz oder das Reaktionsgemisch, und gegebenenfalls der Katalysator (die Katalysatoren), in einen vom Batch-Typ oder diskontinuierlichen Reaktor zugegeben wird, der zum Empfangen von Mikrowellenfrequenzen oder Radiofrequenzen geeignet ist.

13. Verfahren nach irgendeinem der Ansprüche 1 bis 12, **dadurch gekennzeichnet, dass** das Reagenz oder das Reaktionsgemisch, und gegebenenfalls der Katalysator (die Katalysatoren), in einen Reaktor zugegeben wird, der zur kontinuierlichen Durchführung von Reaktionen geeignet ist.

14. Verfahren nach Anspruch 8 oder 9, **dadurch gekennzeichnet, dass** die Frequenzen zwischen ungefähr 30 GHz und ungefähr 300 MHz, zwischen 2,45 GHz oder 915 MHz, zwischen ungefähr 300 MHz und ungefähr 3 MHz und zwischen 13,56 MHz oder 27,12 MHz liegen.

15. Verfahren nach irgendeinem der Ansprüche 1 bis 14, **dadurch gekennzeichnet, dass** die Temperatur, der das Reagenz oder das Reaktionsgemisch und gegebenenfalls der Katalysator (die Katalysatoren), ausgesetzt wird, zwischen 200 und 400 ° C, vorzugsweise zwischen 220 und 350 ° C liegt.

16. Verfahren nach Anspruch 1 bis 15, **dadurch gekennzeichnet, dass** die Temperaturanstiegszeit zwischen 3 und 60 Minuten, vorzugsweise zwischen 3 und 20 Minuten gewählt wird, wenn das Reaktionsgemisch einem dielektrischen Erwärmen unterzogen wird.

17. Verfahren nach irgendeinem der Ansprüche 1 bis 16, **dadurch gekennzeichnet, dass** die Reaktionszeit zwischen 15 Minuten und 15 Stunden, vorzugsweise zwischen 15 Minuten und 360 Minuten, besonders bevorzugt zwischen 15 und 120 Minuten liegt.

18. Verfahren nach irgendeinem der Ansprüche 1 bis 17, **dadurch gekennzeichnet, dass** die Polymerisation in einer sauerstoffreichen oder inerten Atmosphäre, unter vermindertem Druck, vorzugsweise zwischen 50 und 10 mm Quecksilber, bei regelmäßiger Erneuerung der Atmosphäre durchgeführt wird.

19. Verfahren nach irgendeinem der Ansprüche 1 bis 18, **dadurch gekennzeichnet, dass** die Polymerisation gestoppt wird, indem man das Reagenz oder das Reaktionsgemisch auf eine Temperatur unterhalb der Polymerisationstemperatur abkühlen lässt oder abkühlt, und dies entsprechend der Viskosität, die man erhalten möchte.

20. Verfahren nach irgendeinem der Ansprüche 1 bis 19, **dadurch gekennzeichnet, dass** das erhaltene Polymernetzwerk zusätzlichen Behandlungen unterzogen wird.

21. Verfahren nach Anspruch 20, **dadurch gekennzeichnet, dass** die zusätzliche Behandlung ausgewählt ist aus Hydrierung, Verfärbung und Desodorierung, wenn die thermische Behandlung zusätzliche Eigenschaften wie Geruch, Farbe verleiht.

22. Polymernetzwerk hergestellt nach einem Verfahren wie in irgendeinem der Ansprüche 1 bis 21 beschrieben.

23. Verwendung des Polymernetzwerkes nach Anspruch 22 zur Herstellung einer kosmetischen oder dermatologischen Zusammensetzung.

24. Verwendung eines Polymernetzwerkes nach Anspruch 22 als Weichmacher oder Feuchtigkeitsspender.

25. Verwendung eines Polymernetzwerkes nach Anspruch 22 zur Herstellung einer kosmetischen oder dermatologischen Zusammensetzung ausgewählt aus Schaumbädern, Lippenstiften, Cremes, Cremes für trockene Haut, Rasiercremes, After-Shave-Lotionen, Pudern, Seifen, Shampoos, wasserfesten Sonnenschutzmitteln, Make-up-Entfernern, Badeölen, Lippenbalsamen, Salben, Lippenpflegestiften.

26. Verwendung eines Polymernetzwerkes nach Anspruch 22 um die Haut weich zu machen, zu schützen oder um der Austrocknung der Haut vorzubeugen.

27. Verwendung eines Polymernetzwerkes nach Anspruch 22 als Lanolinersatz.

## Claims

1. Process for polymerisation by means of heat treatment of esters of unsaturated fatty acids, alone or in admixture with polyterpenes, **characterised in that** there are added to the reaction mixture from 1 to 6% by weight of the mixture of one or more compounds chosen from glycerol, polyglycerol, sorbitol, monoethanolamine MEA and vitamins C and E.

2. Process according to claim 1, **characterised in that** the esters of unsaturated fatty acids are chosen from oils and fats of plant origin.

3. Process according to claim 2, **characterised in that** the oils of plant origin are chosen from rapeseed oil, sunflower oil, groundnut oil, olive oil, walnut oil, corn oil, soybean oil, linseed oil, safflower oil, apricot kernel oil, sweet almond oil, hemp oil, grapeseed oil, coconut oil, palm oil, cottonseed oil, babassu oil, jojoba oil, sesame oil, argan oil, milk thistle oil, pumpkin seed oil, raspberry oil, Karanja oil, neem oil, poppyseed oil, Brazil nut oil, castor oil, dehydrated castor oil, hazelnut oil, wheatgerm oil, borage oil, evening primrose oil, tung oil, tall oil, which may undergo prior treatment in order to render them more reactive or, conversely, less reactive.

4. Process according to claim 1, **characterised in that** there are used as esters of unsaturated fatty acids, alone or in a mixture, one or more esters obtained by esterification of a monoalcohol and/or polyol and at least one unsaturated fatty acid; waxes; phospholipids; sphingolipids; glucolipids.

5. Process according to claim 4, **characterised in that** the oils and fats of plant origin undergo prior treatment in order to render them more reactive or, conversely, less reactive, chosen from hydrogenation, hydroxylation, epoxidation, phosphitation, sulfonation.

6. Process according to any one of claims 1 to 5, **characterised in that** the process is carried out without a catalyst and in an oxygen-free atmosphere.

7. Process according to any one of claims 1 to 6, **characterised in that** the reaction mixture is subjected to dielectric heating in order to carry out the polymerisation.

8. Process according to claim 7, **characterised in that** heating is carried out by the use of microwave frequencies.

9. Process according to claim 7, **characterised in that** heating is carried out by the use of radio frequencies.

10. Process according to any one of claims 1 to 9, **characterised in that** heterogeneous or homogeneous catalysts are added to the reagent or to the reaction mixture.

11. Process according to any one of claims 1 to 10, **characterised in that** catalysts responding to radio frequencies or to microwave frequencies are added to the reagent or to the reaction mixture.

12. Process according to any one of claims 1 to 11, **characterised in that** the reagent or the reaction mixture, and optionally the catalyst(s), is/are placed in a batch-type or discontinuous reactor adapted to receive microwave frequencies or radio frequencies.

13. Process according to any one of claims 1 to 12, **characterised in that** the reagent or the reaction mixture, and optionally the catalyst(s), is/are placed in a reactor adapted to carry out continuous reactions.

14. Process according to claim 8 or 9, **characterised in that** the frequencies are between approximately 30 GHz and approximately 300 MHz, between 2.45 GHz and 915 MHz, between approximately 300 MHz and approximately 3 MHz and between 13.56 MHz and 27.12 MHz.

15. Process according to any one of claims 1 to 14, **characterised in that** the temperature to which the reagent or the reaction mixture, and optionally the catalysts(s), is/are subjected is between 200 and 400°C, preferably between 220 and 350°C.

16. Process according to claim 1 to 15, **characterised in that** the time in which the temperature is increased is chosen between 3 and 60 minutes, preferably between 3 and 20 minutes, when the reaction mixture is subjected to dielectric heating.

17. Process according to any one of claims 1 to 16, **characterised in that** the reaction time is between 15 minutes and 15 hours, preferably between 15 minutes and 360 minutes, yet more preferably between 15 and 120 minutes.

18. Process according to any one of claims 1 to 17, **characterised in that** the polymerisation is carried out under an oxygen-rich or inert atmosphere; under reduced pressure, preferably between 50 and 10 mm of mercury; while regularly renewing the atmosphere.

19. Process according to any one of claims 1 to 18, **characterised in that** the polymerisation is stopped by allowing the reagent or the reaction mixture to cool, or by cooling the reagent or the reaction mixture, to a temperature below the polymerisation temperature, depending on the viscosity that is to be obtained.

20. Process according to any one of claims 1 to 19, **characterised in that** the polymeric network obtained is subjected to additional treatments.

21. Process according to claim 20, **characterised in that** the additional treatment is chosen from hydrogenation, decolouration and deodorisation if said heat treatment imparts additional properties such as odour, colour.

22. Polymeric network produced by a process as described in any one of claims 1 to 21.

23. Use of the polymeric network according to claim 22 in the preparation of a cosmetic or dermatological formulation.

24. Use of a polymeric network according to claim 22 as an emollient or moisturiser.

25. Use of a polymeric network according to claim 22 in the preparation of a cosmetic or dermatological formulation chosen from foam baths, lipsticks, creams, creams for dry skin, shaving creams, after-shave lotions, powders, soaps, shampoos, waterproof sun creams, make-up removers, bath oils, lip balms, ointments, lip salves.

26. Use of a polymeric network according to claim 22 for softening the skin, protecting the skin or preventing dehydration of the skin.

27. Use of a polymeric network according to claim 22 as a lanolin substitute.
